**Europäisches Patentamt**

**European Patent Office**

**Office européen des brevets**

(19)

(11) Veröffentlichungsnummer : **0 323 397 B1**

(12) # EUROPÄISCHE PATENTSCHRIFT

(45) Veröffentlichungstag der Patentschrift :
**22.01.92 Patentblatt 92/04**

(51) Int. Cl.$^5$ : **A61F 2/06**

(21) Anmeldenummer : **88810753.9**

(22) Anmeldetag : **04.11.88**

(54) **Künstliche Organ- oder Gefässwand.**

(30) Priorität : **07.12.87 CH 4769/87**

(43) Veröffentlichungstag der Anmeldung :
**05.07.89 Patentblatt 89/27**

(45) Bekanntmachung des Hinweises auf die
Patenterteilung :
**22.01.92 Patentblatt 92/04**

(84) Benannte Vertragsstaaten :
**AT DE FR GB IT**

(56) Entgegenhaltungen :
**EP-A- 0 130 401**
**EP-A- 0 216 149**
**EP-A- 0 223 415**
**EP-A- 0 248 247**
**US-A- 4 355 426**

(73) Patentinhaber : **GEBRÜDER SULZER**
**AKTIENGESELLSCHAFT**
**Zürcherstrasse 9**
**CH-8401 Winterthur (CH)**
Patentinhaber : **IMS-BIOPUR AG**
**Südstrasse**
**CH-8807 Freienbach (CH)**

(72) Erfinder : **Bittmann, Peter, Dr.**
**Langackerstrasse 126**
**CH-8704 Herrliberg (CH)**
Erfinder : **Dittes, Peter**
**Hasenweidstrasse 5**
**CH-8635 Oberdürnten (CH)**
Erfinder : **Müller, Werner, Dr.**
**Wannenstrasse 6**
**CH-8542 Wiesendangen (CH)**

## Beschreibung

Die Erfindung betrifft eine künstliche Organ- oder Gefässwand einer bioinerten, membranartigen Kunststoff-Prothese zum einseitigen Beschichten mit einer geschlossenen Lage von epithelialen Zellen, wofür die Wand durchgehende, offene und mindestens teilweise miteinander kommunizierende Mikroporen aufweist, die durch Trennwände voneinander getrennt sind.

Für einen Ersatz kleinlumiger arterieller Blutgefässe, beispielsweise mit Durchmesser kleiner 7 mm sind bereits zahlreiche Konstruktionen bekannt (siehe z.B. US-PS 4,355,426 ; WO 85/03444 und 85/03445 ; EP-A-0130401 und EP-A-0216149). Vorzugsweise bestehen diese Prothesen aus polymeren Elastomer-Werkstoffen, wie z.B. Polyurethan. Aus physiologischen Erfordernissen werden derartige Prothesen innen mit einer geschlossenen Schicht von epithalialen, z.B. Endothel-, Zellen ausgekleidet. Die Prothesenwand ist daher mit offenen miteinander kommunizierenden Mikroporen versehen, die gegebenenfalls mit nach aussen zunehmenden Porengrösse durch die ganze Wanddicke von unter Umständen einigen Millimetern (mm) hindurchgehen können.

Für die den Endothel-Zellen zugewandten innersten Schichten der Gefässwände werden dabei Porengrössen von 0,5 bis 20 Mikrometer (μm) angegeben. In der EP-A-0223415 werden biokompatible Membranen beschrieben, die aus einer ausgewählten Elastomer-Lösung ("Spandex") mittels eines Phasentrennungsverfahrens hergestellt werden, wobei Hautschichten mit Mikroporen entstehen. Mit einem besondern Verfahren ("Example V") lässt sich eine Membran erzeugen, deren Poren in der Hautschicht maximale lineare Abmessungen zwischen 0.1 und 1 μm aufweisen. In der Praxis haben sich bei den bisherigen Konstruktionen verschiedene Nachteile ergeben, besonders, wenn die mit den Endothel-Zellen beschichtete Prothesenwand den Scherkräften eines vorbeifliessenden Blutstromes ausgesetzt worden ist. Einer der Nachteile besteht z.B. darin, dass die epithelialen Zellen keine Strukturen als geschlossene Schicht überwachsen, deren Poren grössen 3 μm sind, da sie in grössere Poren hineinwachsen und diese wie Kapillaren zirkulär auskleiden ; sie bilden dann keine geschlossene Oberflächen-Schicht mehr. Andererseits hat sich herausgestellt, dass glatte Oberflächen mit Abmessungen von mehr als 3 μm Breite oder Länge zwischen den Poren eine sehr schlechte Haftung zur Folge haben. Aufgabe der Erfindung ist es, die bisherigen Konstruktionen von mikroporösen Gefässwänden für künstliche arterielle Blutgefässe derart zu verbessern, dass auf ihnen das Wachsen einer geschlossenen Schicht aus lebensfähigen epithalialen Zellen gewährleistet und andererseits eine verbesserte Langzeithaftung der Zellen auf der Prothesenwand erzielt wird.

Diese Aufgabe wird dadurch gelöst, dass bei der Herstellung der Prothesenwand aus einer Elastomerlösung mittels eines Phasentrennungsverfahrens und bei geeigneter Wahl von Temperatur, Konzentration des Elastomers sowie Zusammensetzung des Lösungsmittels eine poröse Wand entsteht, für welche die Mikroporen auf der von den Zellen zu besiedelnden Oberfläche als Feinstporen mit einer maximalen linearen Abmessung zwischen 0,1 und 3 μm ausgebildet sind, und dass ferner die Dicke der Trennwände zwischen den Porenöffnungen an dieser Oberfläche von 1 μm bis zu maximal 3 μm beträgt.

Einerseits "betrachten" die epithelialen Zellen Poren mit den Maximalabmessungen der geforderten Feinstporen noch nicht als Kapillarröhrchen, in die sie ringförmig einwachsen, sondern überdecken die Poren als geschlossene Schicht. Andererseits bilden diese Feinstporen ein verzweigtes Netz einer löchrigen rauhen Oberfläche, in die, ausgehend von der basalen Zellmembran bzw. der Basallamina, fibrilläre Elemente der subendothelialen Extrazelluläre Matrix (ECM) einwachsen, die als "gebündelte Fasern" eine mechanische Verankerung auf dem Substrat ergeben.

Eine ausreichende Ernährung und Erneuerung dieser fibrillären Elemente ist nur gewährleistet, wenn eine Mindestporengrösse von 0,1 μm gewährleistet wird.

Als vorteilhaft hat es sich erwiesen, wenn die Tiefe des mit den Feinstporen versehenen Bereiches an der Oberfläche zwischen 0,5 und 3 μm beträgt ; die mechanische Verankerung der fibrillären Elemente kann verbessert werden, wenn die Feinstporen mindestens zum Teil innerhalb des Feinstporen-Bereiches miteinander kommunizieren.

Als Materialien für die Gefässwand können Elastomere, wie Styrol-Butadien-Styrol-Blockpolymere und deren Abmischungen mit z.B. Polyurethanen (PUR) oder mit PUR-Polysiloxan-Copolymeren, eingesetzt werden ; ferner auch PUR im speziellen segmentierte PUR, oder PUR-Polysiloxan-Copolymere, Natur- oder Synthesekautschuke oder andere Werkstoffe mit elastomeren Eigenschaften. Die Elastomere werden als 1-70%-ige Lösungen eingesetzt. Als Lösungsmittel kommen aliphatische, aromatische, polare oder unpolare Verbindungen, wie z.B. Xylol, Decahydronaphtalin, N, N-Dimethylacetamid, Dimethylformamid, N -Methylpyrolidon, Ketone, Aldehyde, Alkohole oder deren Abmischungen in Frage.

Die Herstellung einer erfindungsgemässen rohrförmigen porösen Prothesenwand, deren Porengrösse, wie an sich bekannt, nach aussen grösser werden kann, erfolgt nach den bekannten Tauchoder Extrusionsverfahren. Zur Erreichung einer Porenstruktur wird das übliche Phasentrennungsverfahren zur Koagulierung eines Elastomers eingesetzt. Die Porengrösse kann dabei über die bei der Phasentrennung eingehaltene Temperatur, die Konzentration des Elastomers sowie die Zusammensetzung der Lösungsmittel für die Tauchbehandlung und für die Phasentrennung beeinflusst werden.

Als Trennmedium kommen polare und apolare, organische und anorganische Detergenzien, wie z.B. $H_2O$, Alkohole, Aldehyde, Sulphonate, Ethoxi- oder Propoxilate, cyclische oder heterocyclische Kohlenwasserstoffverbindungen, Amine, Amide oder Abmischungen der verschiedenen Verbindungen in Frage.

Weiterhin ist es selbstverständlich möglich, zur Erreichung ein einem natürlichen Gefäss ähnlichen Compliance in bekannter Weise ausserhalb der mit Feinstporen versehenen Tiefe ein Armierungsmaterial in die Prothesenwand einzubauen. Dieses Material kann aus organischen oder anorganischen Werkstoffen als Vlies, Gewirk, Gewebe oder als geformtes Monofil eingesetzt werden. Die Materialen können dabei texturiert oder gestreckt sein.

Für eine Kontrolle und Messung der Porengrössen und Wanddicken haben sich rasterelektronenmikroskopische Verfahren als geeignet erwiesen.

Ausführungsbeispiel

Auf einem Stab mit einigen Millimetern Durchmesser aus einer Chrom-Nickel-Vanadium-Stahllegierung werden nach dem bekannten Tauchverfahren mehrere Schichten einer harnstoffvernetzten Polyurethanäther-Verbindung durch Eintauchen in eine 10%-ige N-Methylpyrolidon-Lösung erzeugt. Zwischen den einzelnen Tauchprozessen findet ein Phasentrennungsprozess mit einer 1 ‰-igen Lösung von Nonylphenolethoxilat in Wasser statt.

Nach dem zweiten Trennungsprozess wird Polyestervlies als Wandverstärkung in die Prothese eingebaut. Anschliessend wird weiter beschichtet bis die gewünschte Wanddicke, die beispielsweise zwischen 0,1 und 1 mm beträgt, erreicht ist.

Schliesslich wird die Prothese von der Matrize gelöst und durch Trocknung und Extraktion von eventuell noch vorhandenen Lösungsmitteln gereinigt und sterilisiert.

**Patentansprüche**

1. Künstliche Organ- oder Gefässwand einer bioinerten, membranartigen Kunststoff-Prothese zum einseitigen Beschichten mit einer geschlossenen Lage von epithelialen Zellen, wofür die Wand durchgehende, offene und mindestens teilweise miteinander kommunizierende Mikroporen aufweist, die durch Trennwände voneinander getrennt sind, dadurch gekennzeichnet, dass bei der Herstellung der Prothesenwand aus einer Elastomerlösung mittels eines Phasentrennungsverfahrens und bei geeigneter Wahl von Temperatur, Konzentration des Elastomers sowie Zusammensetzung des Lösungsmittels eine poröse Wand entsteht, für welche die Mikroporen auf der von den Zellen zu besiedelnden Oberfläche als Feinstporen mit einer maximalen linearen Abmessung zwischen 0,1 und 3 μm ausgebildet sind, und dass ferner die Dicke der Trennwände zwischen den Porenöffnungen an dieser Oberfläche von 1 μm bis zu maximal 3 μm beträgt.

2. Gefässwand nach Anspruch 1, dadurch gekennzeichnet, dass die Tiefe des mit den Feinstporen versehenen Bereiches an der Oberfläche zwischen 0.5 und 3 μm beträgt.

3. Gefässwand nach Anspruch 2, dadurch gekennzeichnet, dass die Feinstporen mindestens zum Teil innerhalb des Tiefenbereiches miteinander kommunizieren.

**Claims**

1. A synthetic organ wall or vessel wall of a bioinert membrane-like plastics prosthesis adapted to be coated on one side with a closed layer of epithelial cells, to which end the wall is formed with continuous open micropores which intercommunicate with one another at least to some extent and which are separated from one another by partitions, characterised in that in the preparation of the prosthesis wall from an elastomer solution by means of a phase separation process and given an appropriate choice of temperature, elastomer concentration and composition of the solvent, a porous well is produced for which the micropores on the surface to be colonised by the cells take the form of very fine pores having a maximum linear dimension of from 0.1 to 3 μm, and the thickness of the partitions between the pore apertures on the latter surface is from 1 μm to at most 3 μm.

2. A vessel wall according to claim 1, characterised in that the depth of the fine-pored zone on the surface is between 0.5 and 3 μm.

3. A vessel wall according to claim 2, characterised in that the very fine pores communicate with one another at least to some extent in the depth zone.

**Revendications**

1. Paroi artificielle d'organe ou de vaisseau d'une prothèse en forme de membrane de matière plastique biologiquement inerte, destinée à être revêtue d'un côté d'une couche fermée de cellules épithéliales, la paroi comportant à cette fin des micropores continus, ouverts, communiquant au moins partiellement les uns avec les autres et séparés les uns des autres par des cloisons, caractérisée en ce que la paroi de la prothèse est réalisée à l'aide d'une solution d'élastomère par un procédé de séparation des phases et un choix convenable de la température, de la concentration de l'élastomère ainsi que de la composition du solvant permet la formation d'une paroi poreuse dont les micropores de la surface devant être colonisée par les cellules consistent en des pores extrêmement fins ayant une dimension linéaire maximale comprise entre 0,1 et 3 μm et, par ailleurs, en ce que l'épaisseur des cloisons situées entre les ouvertures des pores à cette surface est comprise entre 1 μm et au maximum 3 μm.

2. Paroi de vaisseau selon la revendication 1, caractérisée en ce que la profondeur de la région de la surface comprenant les pores extrêmement fins est comprise entre 0,5 et 3 μm.

3. Paroi de vaisseau selon la revendication 2, caractérisée en ce que les pores extrêmement fins communiquent au moins en partie les uns avec les autres à l'intérieur de la région ayant ladite profondeur.